# EUROPEAN PATENT SPECIFICATION

(11) **EP 0 914 456 B1**
(45) Date of publication and mention of the grant of the patent: **22.03.2006**
(21) Application number: 97920819.6
(22) Date of filing: 02.05.1997
(51) Int. Cl.: C12N 15/86, C12N 15/11, C12N 5/10, A61K 48/00

(54) **GENE EXPRESSION IN MONOCYTES AND MACROPHAGES**
GENEXPRESSION IN MONOCYTEN UND MAKROPHAGEN
EXPRESSION DE GENES DANS DES MONOCYTES ET DES MACROPHAGES

(30) Priority: 02.05.1996 GB 9609261
(43) Date of publication of application: 12.05.1999
(73) Proprietor: GLAXO GROUP LIMITED, Greenford, Middlesex UB6 ONN (GB)
(72) Inventor: GREAVES, David, Robert, Oxford OX1 3RE (GB)
(74) Representative: Rees, Marion Lindsay
(86) International application number: PCT/GB1997/001209
(87) International publication number: WO 1997/042337

(56) References cited:
- WO-A-94/21806
- WO-A-95/08635
- WO-A-95/33841
- BRISTOL-MYERS SQUIBB CANCER SYMP. (1993), 15(APPLICATION OF BASIC SCIENCE TO HEMATOPOIESIS AND TREATMENT OF DISEASE), 21-38 CODEN: BMSSE7, 1993, XP002037064 GROSVELD, FRANK ET AL: "The regulation of the human.beta.-globin locus"
- HOLNESS, CLAIRE ET AL.: "Molecular cloning of CD68, a human macrophage marker related to lysosomal glycoprotein" BLOOD, vol. 81, 1993, pages 1607-1613,

## Description

The present invention relates to the regulatory nucleotide sequences associated with the CD68 gene.

### Background to the Invention

### Locus Control Regions

Mammallan gene expression is regulated through cis- linked DNA sequences. Promoter sequences lie immediately 5' of the gene's transcription initiation site and enhancer sequences can lie within or close to the gene which they regulate. In 1987 it was shown that DNA sequences found within 70 kilobases (kb) 5' of the human. β- globin gene on human chromosome 11 were able to effect high-level, copy number dependent expression of the human β-globin gene in erythroid cells of transgenic mice (1) Grosveld, F., *et al*. Cell, 1987, 51, 975-985. These DNA sequences which play a key role in the in vivo regulation of globin gene expression were termed Dominant Control Sequences (DCR) - later renamed Locus Control Regions (LCRs). Locus Control Regions have been described for other red cell genes such as the genes of the human α-globin locus (2) Greaves, D.R., *et al* Cell, 1989, **56**, 979-986 and LCRs have been described which direct high level gene expression in other cell types. Examples include the human CD2 gene which has a T-cell specific LCR and the 3' end of the Ca immunoglobulin heavy chain locus which directs high level expression in B-cells (3) Madisen, L and Groudine, M. Genes and Development, 1994, B, 2212-2226. WO94/21806 discloses serveral Locus. Control Regions, such as CD2 or macrophage LCR sequences, and their use to regulate tissue specific gene expression. The CD68 gene is expressed in all macrophage cells. The specificity of expression in vivo is high. The origin of this specificity has been investigated and surprisingly it has been found that small regions of the CD68 gene are responsible.

### Summary of Invention

The present invention therefore provides a cloned or isolated polynucleotide having the function of a transcriptional regulatory sequence (trs) and comprising:
(a) a polynucleotide fragment having at least 70% identity to the polynucleotide of Seq ID No. 2;
(b) a polynucleotide which is complementary to the polynucleotide of (a); or
(c) a polynucleotide comprising at least 15 sequential bases of the polynucleotide of (a) or (b).

Preferably the polynucleotide fragment has at least 80% identity to the polynucleotide of Seq ID No. 2, more preferably at least 90% identity to the polynucleotide of Seq ID No. 2. Most preferably the isolated polynucleotide according to the invention comprises the polynucleotide of Seq ID No.2.

The present invention further provides an isolated polynucleotide comprising the transcriptional regulatory sequence of CD68.

The present invention additionally provides an isolated polynucleotide comprising the transcriptional regulatory sequence of CD68 and a polynucleotide operatively linked thereto encoding a heterologous polypeptide.

The present invention also provides a vector comprising a polynucleotide as defined herein and a host cell comprising the vector.

The present invention further provides a process for producing a polypeptide which process comprises transforming or transfecting a cell with a vector as defined herein such that the cell expresses the polypeptide encoded.
In a further aspect this invention results from the discovery of DNA sequences exhibiting the properties of a locus control region associated with the CD68 gene. In one aspect the invention provides a vector for the integration of a gene into the genetic material of a mammalian host cell such that the gene may be expressed in the host cell, the vector comprising a promoter and the said gene and a Locus Control Region capable of eliciting host cell-type restricted, integration site independent, copy number dependent expression of said gene, characterised in that the Locus Control Region is located within a region extending from 14kb upstream to 25 kb downstream of the CD68 gene.

### Functional definition of an LCR

Locus Control Regions are fundamentally different from other gene regulatory sequences in that they are not subject to position effects after integration into host cell chromosomes. Work with the human β-globin gene LCR revealed the three criteria which distinguish this class of DNA sequence from other regulatory sequences such as promoters and enhancers (1,4,5). Grosveld, F., *et al* Cell, 1987, **51,** 975-985., Blom van Assendelft, M., *et al*, Cell, 1989, **56**, 969-977., Talbot, D., *et al*. Nature, 1989, **338**, 352-355.
1) LCRs direct position-independent expression of co-linked genes after integration into host genomes either in transgenic animals or in cell lines. As a consequence all transgenic animals carrying an intact copy of the transgene will express the transgene at a significant level. This is in sharp contrast to results obtained with other DNA sequences.
2) LCRs exhibit strict tissue specificity in the pattern of transgene expression. A human β-globin gene placed downstream of a human β- globin LCR is expressed only in red cells of transgenic mice (1, 4). The exact same human β-globin gene fragment placed downstream of a human CD2 LCR is expressed at high levels in all T-cells but not red cells of transgenic animals (2). Greaves, D.R., *et al.* Cell, 1989, 56, 979-986.
3) LCRs direct high-level, copy number-dependent gene expression. Human β-globin transgenes can be expressed as efficiently on a per copy basis as endogenous murine β-globin genes in the erythroid cells of transgenic mice. When each transgene expresses at the same high level this leads to a direct relationship between transgene expression and transgene copy number.
LCR's are thus expected to have a region providing chromatin opening activity, which region provides at least the first and third activities described above. An LCR generally contains at least one transcriptional regulatory sequence such as a promoter or enhancer in addition to the sequence which provides chromatin opening activity.

The genetic diseases β- thalassaemia and sickle cell anaemia are caused by mutations within the human β- globin gene locus. All the red blood cells of the body are derived from a limited number of haematopoietic stem cells found in the bone marrow. The demonstration that the β-globin LCR was able to direct red cell-specific expression regardless of its site of integration into the genome lead us to propose the idea of somatic gene therapy for β- thalassaemia and sickle cell anaemia by introducing, β-globin LCR vectors into haematopoietic stem cells and using such transfected cells in bone marrow transplantation.

This is described in one of the proposed applications set out in the original β-globin LCR patents filed in 1987 and 1989 (UK patents 8718779 & 8904009.1).

The Locus Control Region (LCR) of the invention may be located within a region extending from 5.5 kb upstream to 12 kb downstream of the CD68 gene; particularly from 2940 base pairs (bp) upstream of CD68 gene to 335 bp downstream (shown in Seq ID No. 3) and more particularly within a 3 kb BstX1-BstX1 locus immediately upstream of the CD68 gene. The LCR may be a single continuous sequence or may consist of two or more such sequences linked together with or without intervening polynucleotides. The LCR may consist of, be derived from, or correspond to one or more DNAse hypersensitive sites. If the LCR of the naturally occurring gene locus comprises two or more discrete sub-sequences separated by intervening non-functional sequences (for example, two or more hypersensitive sites) the vector of the invention may comprise an LCR comprising two or more of the sub-sequences linked together with all or part of the intervening sub-sequences removed.

The term "vector" as used herein connotes in its broadest sense any recombinant DNA material capable of transferring DNA from one cell to another.

In another aspect the invention provides a mammalian host cell transformed with a vector as defined. The mammalian host cell is selected from macrophages, monocytes and dendritic cells and their precursors.

In other aspects the invention provides: a method of producing a polypeptide by culturing a mammalian host cell as defined; a method of modifying mammalian host or stem cells by transformation with a vector as defined; transformed mammalian host or stem cells for use in the treatment of a diseased condition in a human or animal body; and use of a vector as defined, or mammalian host or stem cells as defined, for the manufacture of a medicament for the treatment of a disease condition of the human or animal body caused by a gene deficiency.

### Figures

Figure 1- restriction maps of cosmids containing the human CD68 gene.
Figure 2 - DNA sequence of the 5' flanking regions of the human CD68 gene. (shown also in Seq ID No.1)
Figure 3 - Northern blot analysis of RNA expression in stably transfected RAW cells.
Figure 4 - RT PCR analysis of CD68 and macrosialin RNAs in stably transfected RAW cells.
Figure 5 - Northern blot analysis of RNA expression in stably transfected RAW and A20 cells
Figure 6 - RT PCR analysis of CD68 and HPRT RNAs in stably transfected RAW and A20 cells.

### Detailed Description

According to the present invention CD68 genes include human CD68, mammalian non-human CD68, for example mouse, dog, cat, rabbit, pig, cow, horse or rat CD68, or non-mammalian CD68. Mouse CD68 is known as macrosialin. Preferably the CD68 is human.

The nucleotide sequences which regulate transcription of CD68 gene are contained within the COSMID CD68C1 which is obtainable from a commercially available human genomic DNA cosmid library (Stratagene).

A transcriptional regulatory sequence (trs) generally comprises at least one promoter region and optionally at least one enhancer region. A trs may be a single continuous nucleotide sequence or may consist of two or more such sequences linked together with or without intervening polynucleotides. Trs regions are generally 5' (upstream) from the coding region but may also be found 3' (downstream) of the ATG start codon, for example in regions of coding sequence or in an intron. Specifically regions of human CD68 trs have been identified in the first intron downstream of the ATG start codon.

It the trs of the naturally occurring gene locus comprises two or more discrete sub-sequences separated by intervening non-functional sequences (for example, two or more super hypersensitive sites) the vector of the invention may comprise a trs comprising two or more of the sub-sequences linked together with all or part of the intervening sub-sequences removed.
The vector of the invention may be used to integrate into the genome an expression cassette which comprises a CD68 trs and a polynucleotide operatively linked thereto encoding a heterologous polypeptide. The vector may also be used to integrate a chimearic gene.

In either case the open reading frame or coding sequence of the gene or cassette is expressed in the host cell.

The vector comprises at least a trs and an open reading frame. An open reading frame may comprise introns. An open reading frame may comprise only coding regions.

A chimearic gene comprises at least a trs and an open reading frame. Optionally a chimearic gene will further comprise polynucleotide regions which regulate replication, transcription or translation or any other process important to the expression of the polynucleotide in a host cell.

The position of sequences relative to the CD68 gene is generally measured relative to the ATG start codon for upstream regions and relative to the stop codon for downstream regions.

Isolated or cloned means separate "by the hand of man" from its natural state; *i*,*e*., that, if it occurs in nature, it has been changed or removed from its original environment, or both. For example, a naturally occurring polynucleotide or a polypeptide naturally present in a living organism in its natural state is not "isolated," but the same polynucleotide or polypeptide separate from the coexisting materials of its natural state is "isolated", as the term is employed herein. As part of or following isolation, such polynucleotides can be joined to other polynucleotides, such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. Introduced into host cells in culture or in whole organisms, such DNAs still would be isolated, as the term is used herein, because they would not be in their naturally occurring form or environment. Similarly, the polynucleotides and polypeptides may occur in a composition, such as media formulations, solutions for introduction of polynucleotides or polypeptides, for example, into cells, compositions or solutions for chemical or enzymatic reactions, for instance, which are not naturally occurring compositions, and, therein remain isolated polynucleotides or polypeptides within the meaning of that term as it is employed herein.

Expression cassettes themselves are well known in the art of molecular biology. Such an expression cassette contains all essential DNA sequences required for expression of the heterologous enzyme in a mammalian cell. For example, a preferred expression cassette will contain a molecular chimaera containing a coding sequence an appropriate polyadenylation signal for a mammalian gene (i.e., a polyadenylation signal that will function in a mammalian cell), and enhancers and promoter sequences in the correct orientation.

Normally, two DNA sequences are required for the complete and efficient transcriptional regulation of genes that encode messenger RNAs in mammalian cells: promoters and enhancers. Promoters are generally located immediately upstream (5') from the start site of transcription. Promoter sequences are required for accurate and efficient initiation of transcription. Different gene-specific promoters reveal a common pattern of organisation. A typical promoter includes an AT-rich region called a TATA box (which is located approximately 30 base pairs 5' to the transcription initiation start site) and one or more upstream promoter elements (UPEs). The UPEs are a principle target for the interaction with sequence-specific nuclear transcriptional factors. The activity of promoter sequences is modulated by other sequences called enhancers. The enhancer sequence may be a great distance from the promoter in either an upstream (5') or downstream (3') position. Hence, enhancers operate in an orientation- and position-independent manner. However, based on similar structural organisation and function that may be interchanged, the absolute distinction between promoters and enhancers is somewhat arbitrary. Enhancers increase the rate of transcription from the promoter sequence. It is predominantly the interaction between sequence-specific transcriptional factors with the UPE and enhancer sequences that enable mammalian cells to achieve tissue-specific gene expression. The presence of these transcriptional protein factors (tissue-specific, trans-activating factors) bound to the UPE and enhancers (cis-acting, regulatory sequences) enables other components of the transcriptional machinery, including RNA polymerase, to initiate transcription with tissue-specific selectivity and accuracy.

Identity, as known in the art, is the relationship between two or more polynucleotide sequences, as determined by comparing the sequences. In the art, identity also means the degree of sequence relatedness between polynucleotide sequences, as the case may be, as determined by the match between strings of such sequences. Identity can be readily calculated (*Computational Molecular Biology,* Lesk, A.M., ed., Oxford University Press, New York; 1988; *Biocomputing: Informatics and Genome Projects,* Smith, D.W., ed., Academic Press, New York, 1993; *Computer Analysis of Sequence Data,* Part I, Griffin, A.M., and Griffin, H.G., eds., Humana Press, New Jersey, 1994; *Sequence Analysis in Molecular Biology,* von Heinje, G., Academic Press, 1987; and *Sequence Analysis Primer*, Gribskov, M. and Devereux, J., eds., M Stockton Press, New York, 1991). While there exist a number of methods to measure identity between two polynucleotide sequences, the term is well known to skilled artisans (*Sequence Analysis in Molecular Biology*, von Heinje, G., Academic Press, 1987; *Sequence Analysis Primer*, Gribskov, M. and Devereux, J., eds., M Stockton Press, New York, 1991; and Carillo, H., and Lipman, D., SIAM *J. Applied Math*., *48*: 1073 (1988). Methods commonly employed to determine identity between sequences include, but are not limited to those disclosed in Carillo, H., and Lipman, D., SIAM *J*. *Applied Math*., 48:1073 (1988). Preferred methods to determine identity are designed to give the largest match between the sequences tested. Methods to determine identity are codified in computer programs.
Preferred computer program methods to determine identity between two sequences include, but are not limited to, GCG program package (Devereux, J., et al., *Nucleic Acids Research 12(1):* 387 (1984)), BLASTP, BLASTN, and FASTA (Atschul, S.F. et al., *J. Molec. Siol. 215:* 403 (1990)).

Polynucleotides which have 70% or more identity with the polynucleotides herein defined may differ from the defined sequences by virtue of at least one nucleotide substitution, addition, deletion, fusion or truncation in the polynucleotide.

One of the regions in the CD68 gene which has been found to be critical for the macrophage specificity of the CD68 gene is the region which extends from the ATG start codon to the nucleotide approximately 80 base pairs upstream (5') to the ATG start codon. This will be referred to as the -80bp region.

A second region which has been found to be critical is the region downstream (3') of the ATG start codon of the CD68 gene and which contains the first PU.1 site downstream of the start codon. A PU.1 site is a DNA sequence capable of binding the etf - family transcription factor PU.1 which is expressed in macrophages, neutrophills and B-cells.

The first PU.1 site downstream of the start codon is comprised within an intron. In human CD68 the intron containing the first PU.1 site is from nucleotide 32 to nucleotide 137 downstream of the start codon.

The region containing the first downstream PU.1 site and the -80bp region when operatively linked provide macrophage specificity. Seq. ID No 2 comprises the sequence of human CD68 from -80bp to the ATG start codon and comprises a PU.1 site and the first intron downstream of the ATG start codon.

A further region which has been found to be critical in providing specificity is the region containing the PU.1 site approximately 110bp upstream of the ATG start codon. Thus the nucleotide sequence from the ATG start codon to approximately 150 base pairs upstream from the ATG start codon confers enhanced macrophage specific expression.

A further region which confers specificity is at the PU.1 site which is found approximately 432 base pairs upstream from the ATG start codon. Thus the nucleotide sequence which extends from the ATG start codon to approximately 460 base pairs upstream from the start codon confers further enhanced macrophage specific expression.

High macrophage specificity is observed when a gene is expressed under the control of any of the above sequences. High macrophage specificity may also be obtained when a gene is expressed under the control of the region which extends from 2940 base pairs upstream of the ATG start codon to 335 base pairs downstream of the TGA stop codon.

The present invention therefore provides a polynucleotide comprising
(a) a polynucleotide fragment having at least 70% identity to a polynucleotide as defined herein;
(b) a polynucleotide which is complementary to the polynucleotide of (a); or
(c) a polynucleotide comprising at least 15 sequential bases of the polynucleotide of (a) or (b)

Preferably the polynucleotide fragment has at least 80% identity to a polynucleotide as herein defined, more preferably at least 90% identity to the polynucleotide as herein defined. Most preferably the isolated polynucleotide according to the invention comprises the polynucleotide as herein defined.

Specifically the present invention provides a polynucleotide comprising an isolated polynucleotide comprising:
(a) a polynucleotide fragment having at least 70% identity to the polynucleotide of Seq ID No. 3;
(b) a polynucleotide which is complementary to the polynucleotide of (a); or
(c) a polynucleotide comprising at least 15 sequential bases of the polynucleotide of (a) or (b).

A preferred nucleotide according to the invention comprises the region containing the first PU1 site and the -80bp region.

A further preferred nucleotide comprises the region from +150 bp to -137 bp relative to the ATG start codon.

A still further preferred nucleotide comprises the region from +460 bp to -137 bp relative to the ATG start codon.

The nucleotide sequence of each of these regions may be determined from Seq. ID No 3 which shows the sequence of cosmid CD68C1 from 2951bp 5' of the CD68 ATG initiation codon (shown in bold type) to 2090 bp 3' of the CD68 TGA termination codon (shown in bold type). Intron sequences are in lowercase, and CD68 coding and 3' untranslated regions are underlined.

Each of the nucleotide sequences of the invention may be used in inverted form.

More than one copy, for example two, three or four copies, of each of the regions described above may be used to control expression of a single gene. When more than one copy of a region is used, the copies are preferably operably linked. The regions may be used in combination or separately.

The sequence ID No. 2 may be used as a probe to locate the sequence ID No. 3, which may then be manipulated according to conventional techniques using known restriction sites.

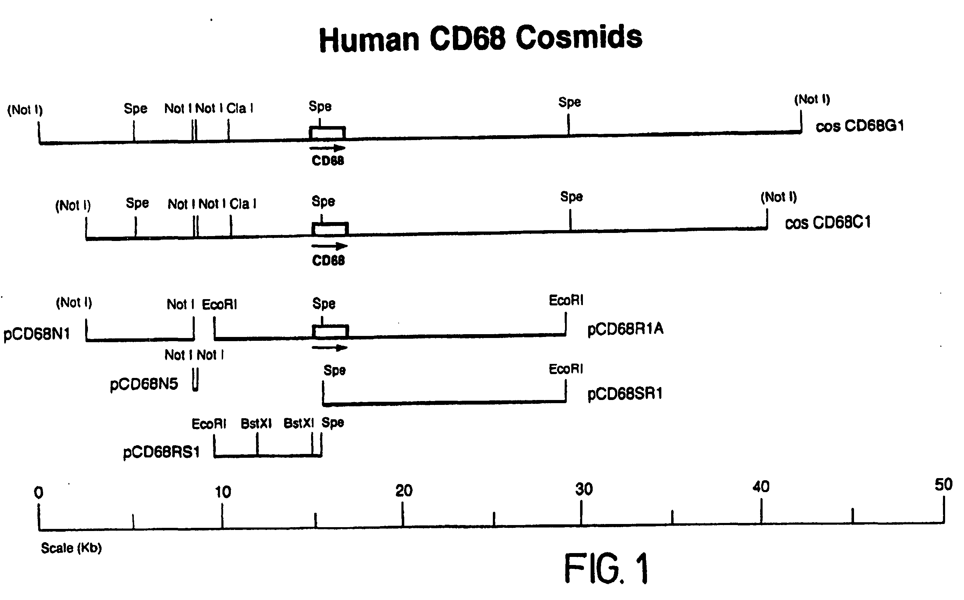

In a search for macrophage-specific gene regulatory sequences recombinant cosmids were isolated containing the human CD68 gene by PCR screening of a human genomic DNA library in the cosmid vector pWE15. Human CD68 is the human homologue of mouse macrosialin, a protein which is found in the phagosomal compartment of all monocytes and macrophages. Two types of human CD68 cosmids were obtained which contain the complete CD68 gene with 14 kilobases of 5' flanking sequences and 25 kilobases of 3' flanking sequences (Figure 1).

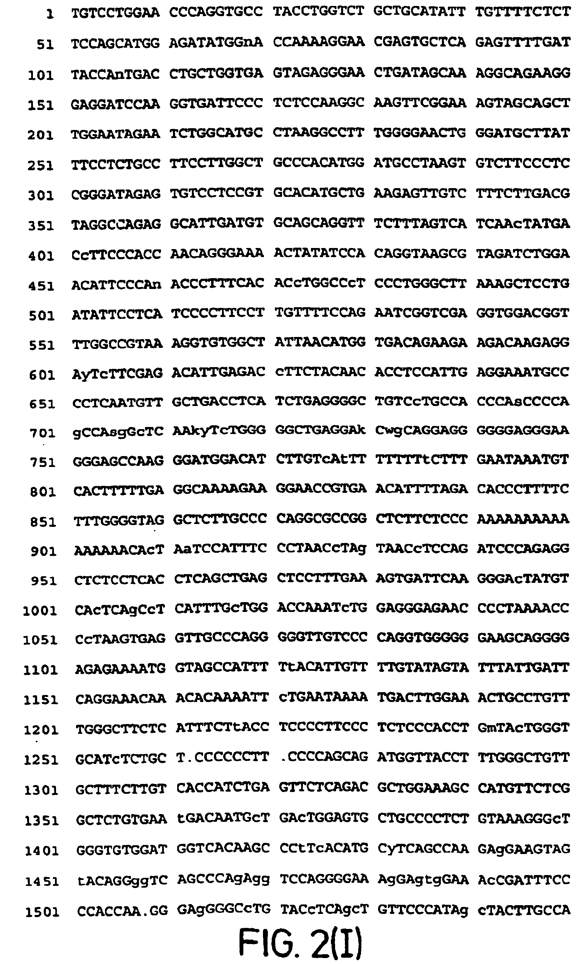

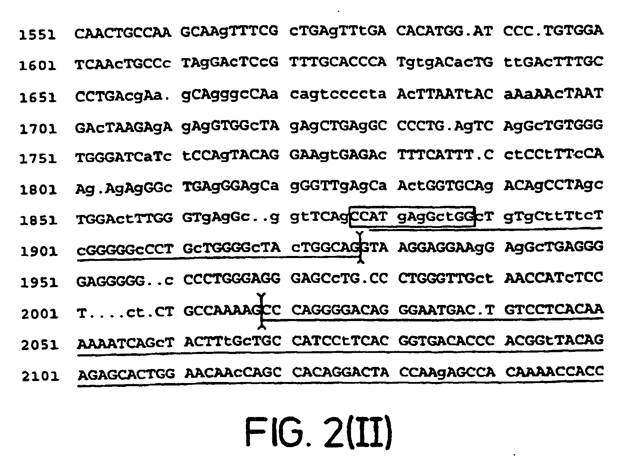

The cosmids were used as probes to demonstrate that the human CD68 gene is located on the short arm of human chromosome 17. The cosmids were used as templates to determine the DNA sequence of the CD68 gene and 1870bp of the CD68 promoter (Figure 2).

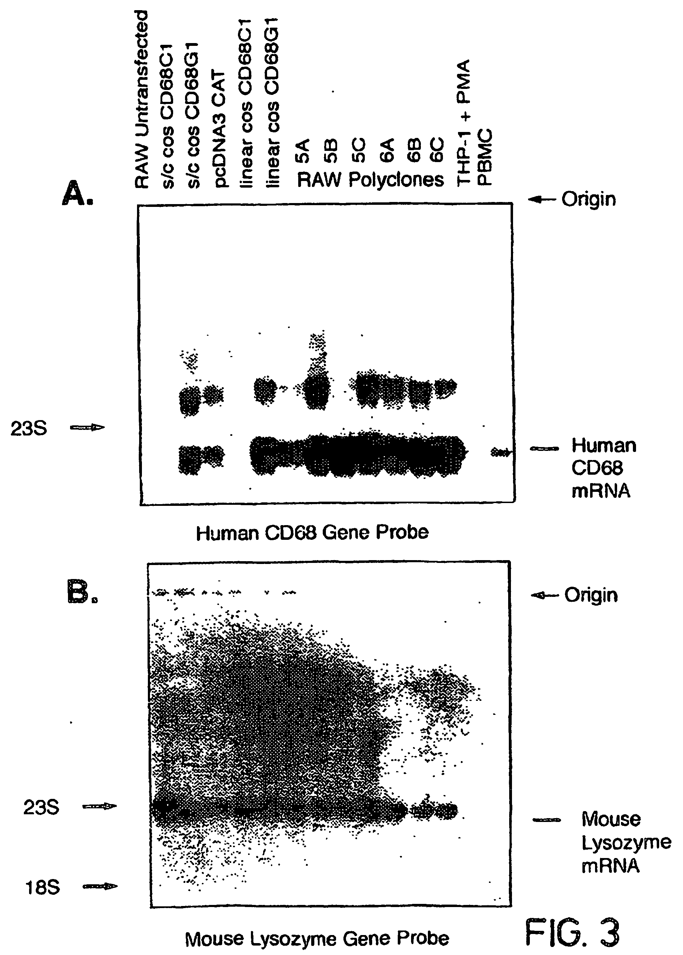

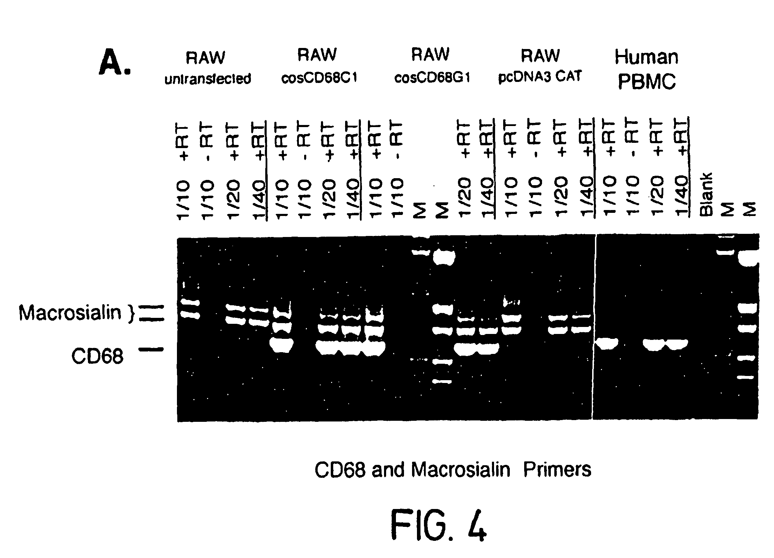

The CD68 cosmids were transfected into the murine macrophage cell line RAW264.7 and stably transfected RAW cell populations and polyclones were selected by growth in G418. Northern blot analysis of total RNA isolated from G418 resistant RAW cells was performed using a radiolabelled human CD68 gene probe (Figure 3A) and a mouse lysozyme probe to control for RNA loading and transfer (Figure 3B). Total RNA prepared from human peripheral blood mononuclear cell (PBMC) cultures was analysed on the same fitter. The human CD68 cosmid transfected RAW cells express very high levels of human CD68 mRNA of the expected size. The levels of human CD68 mRNA are higher in cosmid transfected RAW cells than in cultured human monocytes. This result was confirmed by analysis of transfected RAW cell RNA by Reverse Transcription - Polymerase Chain Reaction (RT-PCR) analysis using human CD68 and murine macrosialin specific PCR primers (Figure 4). The transfected human CD68 gene is expressed at higher levels than the endogenous mouse macrosialin gene.

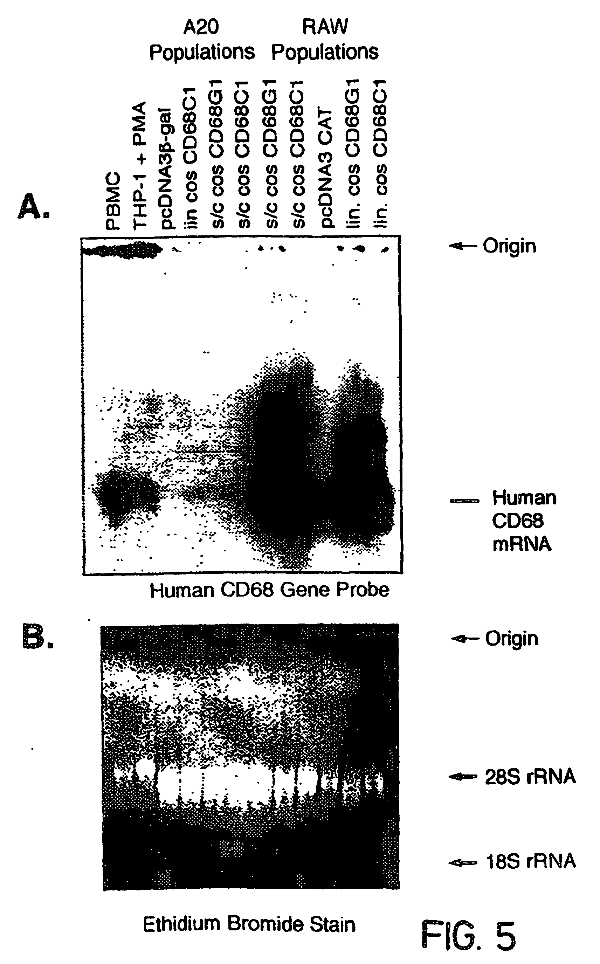

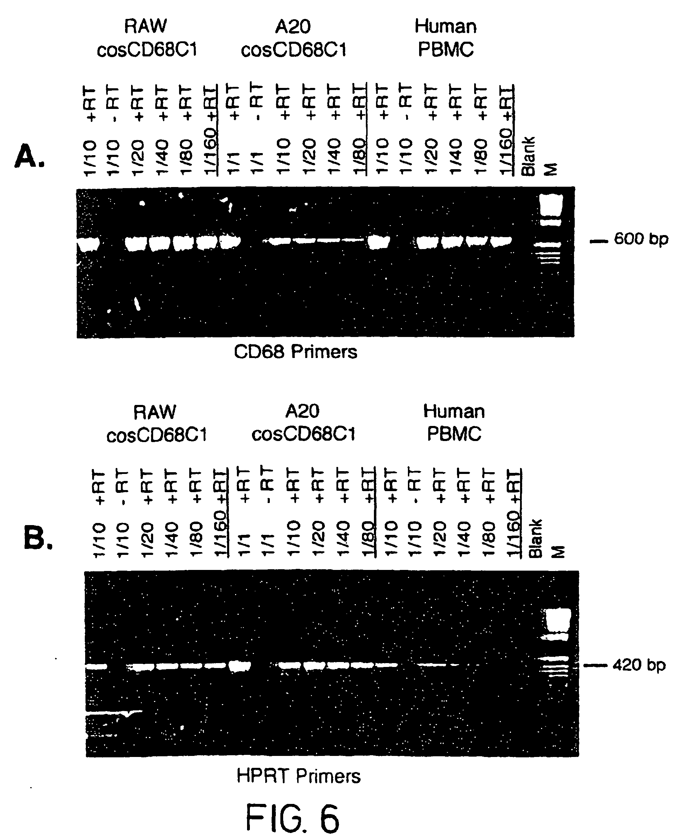

The same CD68 cosmid DNAs were transfected into murine A20 B- cells an d 3T3 fibroblasts. Northern blot analysis of CD68 RNA expression showed levels of CD68 mRNA at least 50 times lower than that observed in murine macrophage cell line RAW264.7 transfected with the same cosmids (Figure 5). These results were confirmed by RT-PCR analysis of transfected cell RNA using CD68 and HPRT specific PCR primers (Figure 6).

Single cell clones from G418 resistant RAW cell populations have been isolated and will be analysed to determine if there is a direct relationship between transgene copy number and transgene expression in transfected RAW264.7 cells which would be predicted if there is a macrophage-specific Locus Control Region in the CD68 cosmids.

The extremely high level of human CD68 mRNA seen in cosmid transfected RAW cells (Figures 3,4 and 5) show that important macrophage-specific genetic regulatory elements are contained within the CD68 cosmids cosCD68C1 and cosCD68G1. Our previous work using the human lysozyme promoter in transgenic animal experiments has shown an important role for intervening sequences and poly A+ addition sequences for transgene expression in macrophages (7,8) Dighe, A.S., *et al*. Immunity, 1995, **3**, 657-666. and Clarke, S., *et al* Proc.Natl. Acad. Sci. (USA), 1996, **93;** 1434-1438.. A role for intervening sequences in ensuring efficient transgene expression has been described in a number of other systems.

### Development of a macrophage-specific gene expression vector

In order to express heterologous genes at a high level in monocytes and macrophages it would be desirable to design a CD68 expression vector. Such a vector should include all the DNA sequences in the human CD68 cosmids which direct high-level, macrophage-specific gene expression along with the CD68 introns and poly A+ sequences and unique restriction enzyme recognition sites for the insertion of cDNAs encoding heterologous genes of interest.

To facilitate manipulation of the human CD68 gene sequences present in cosmid cosCD68C1 a 20kb EcoRI fragment which contains the complete human CD68 gene along with 5.5kb of 5' flanking and 12.5kb of 3' flanking sequences was cloned into the unique EcoRI site of the plasmid vector Bluescript SK- (Stratagene) to give the recombinant plasmid pCD68R1A (Figure1). Two further recombinant plasmids were derived from pCD68R1A which contain the 5' flanking and 3' flanking sequences of the human CD68 gene, pCD68RS1 and pCD68SR1 (Figure 1). In order to engineer the insertion of cDNAs encoding heterologous genes immediately downstream of the CD68 gene promoter and immediately upstream of the CD68 gene's ATG initiation codon the plasmid pCD68RS1 was digested with the restriction enzyme BstX1 and a 3kb BstX1 fragment was cloned into the plasmid vector Bluescript SK(Stratagene) after treatment with T4 DNA polymerase which removes the CD68 ATG initiation codon (Figure 2). The human CD68 genomic DNA fragments present in the recombinant plasmids shown in Figure 1 allow for the development of a versatile and easily manipulated human CD68 expression system for use in macrophage cell lines, transgenic animals and human primary cells.

### Table 1 Promoter activity of CD68 promoter 5' deletion series in transiently transfected RAW264.7 and P388.D1 cells.

RAW264.7 or P388.D1 cells were electroporated in the presence of 20µg of the indicated CAT reporter plasmids and 5µg of the β-galactosidase reporter plasmid pcDNA3 β-gal. Forty eight hours post transfection cell lysates were assayed for β-galactosidase and CAT enzyme activity as described in Experimental Procedures. Cell lysate CAT enzyme activities were corrected for transfection efficiency and the data is expressed as a percentage of the CAT enzyme activity obtained with the SV40 promoter/enhancer plasmid pCATControl in the same experiment. All cell lysate CAT enzyme activities were within the linear range of the assay as determined from a CAT enzyme dilution series analysed in the same experiment. The data shown are from a single transfection experiment and the relative promoter activities were reproducible in at least three independent transfection experiments with each construct in both cell lines.

### Table 2 Comparison of myeloid gene promoter activities in transiently transfected murine macrophage cell lines.

P388.D1 or RAW264.7 cells were electroporated in the presence of 20µg of the indicated CAT reporter plasmids and 5µg of the β-galactosidase reporter plasmid pcDNA3 β-gal. Twenty four hours post transfection cell lysates were assayed for β-galactosidase and CAT enzyme activity as described in Experimental Procedures. Cell lysate CAT enzyme activities were corrected for transfection efficiency and expressed as a percentage of the CAT enzyme activity obtained with the SV40 promoter/enhancer plasmid pCATControl in the same experiment. All cell lysate CAT enzyme activities were within the linear range of the assay as determined from a CAT enzyme dilution series analysed in the same experiment. The data shown are from a single transfection experiment and the promoter activities were reproducible in at least two independent transfection experiments with each construct.

**Table 3**

| Plasmid | Gene | Promoter | Accession No |
|---|---|---|---|
| -2940CD68pCAT | hCD68 | -2940 to +2* | This paper |
| hLZMpCAT | hLysozyme | -517 to +26 | X57103 |
| mLZMpCAT | mLysozyme | -487 to +11 | D13263 |
| CD11b pCAT | hCD11b | -1706 to+91 | M76724 |
| c-fes pCAT | h c-fes | -446 to +71 | X06292 |
| hMSRpCAT | h MSR | -487 to +11 | D13263 |

Table 3 shows Myeloid promoters used in this study

All myeloid gene promoter fragments were cloned into the multiple cloning site of the CAT reporter vector pCATBasic (See Experimental Procedures). Human genes are denoted by the prefix h and murine genes by the prefix m.

Promoter coordinates shown are taken from the given Genbank accession numbers and the major transcription initiation site is denoted as +1 except for

CD68 where the A of the ATG translation initiation codon is denoted as +1 (*).

### Table 5 The effect of the CD68 first intron in transiently transfected RAW264.7 and CHO cell lines.

RAW264.7 cells were electroporated in the presence of 20µg of the indicated CAT reporter plasmids and 5µg of the β-galactosidase reporter plasmid pcDNA3 β-gal. Forty eight hours post transfection transfected cell lysates were assayed for β-galactosidase and CAT enzyme activity. CHO cells were transfected with 4.5µg of the indicated CAT reporter plasmids and 0.5µg of the β-galactosidase reporter plasmid pcDNA3 β-gal complexed with 50µg of the cationic lipid Lipofectamine (Gibco BRL). Cell lysate CAT enzyme activities were corrected for transfection efficiency and expressed as a percentage of the CAT enzyme activity obtained with the SV40 promoter/enhancer plasmid pCATControl analysed in the same experiment. All cell lysate CAT enzyme activities were within the linear range of the assay as determined from a CAT enzyme dilution series analysed in the same experiment. The data shown are from a single transfection experiment and the promoter activities were reproducible in at least three independent transfection experiments with each construct in each cell line.

### Table 4 The effect of the CD68 first intron on CD68 and HIV promoters

RAW264.7 and P388.D1 cells were electroporated in the presence of 20µg of the indicated CAT reporter plasmids and 5µg of the β-galactosidase reporter plasmid pcDNA3 β-gal. Forty eight hours post transfection transfected cell lysates were assayed for β-galactosidase and CAT enzyme activity. Cell lysate CAT enzyme activities were corrected for transfection efficiency and expressed as a percentage of the CAT enzyme activity obtained with the SV40 promoter/enhancer plasmid pCATControl analysed in the same experiment. All cell lysate CAT enzyme activities were within the linear range of the assay as determined from a CAT enzyme dilution series analysed in the same experiment. The data shown are from a single transfection experiment and the promoter activities were reproducible in at least three independent transfection experiments with each construct in each cell line.

### METHODS

### Construction of promoter reporter plasmids

A 2940bp BstXI fragment was purified from an EcoRl - Spel fragment subcloned from cosCD68C1. The BstXI fragment was rendered blunt ended by incubation with T4 DNA polymerase and all four dNTPs and cloned into the EcoRV site of pBluesapt SK- (Stratagene) to give plasmid pCD68Bst3-2. The 3' BstXI site contains the CD68 ATG initiation codon which was removed by the 3' exonuclease activity of T4 DNA polymerase. A Hind III - Xbal fragment containing 2940 bp of DNA upstream of the CD68 ATG codon was cloned into the reporter vector pCAT Basic (Promega, Genbank accession number X65322) to give the plasmid -2940 CD68pCAT. Plasmid -2940 CD68pCAT was digested with XhoI, BglII or SstI and overhanging ends filled in by treatment with the Klenow fragment of DNA polymerase or T4 DNA polymerase before ligation of phosphorylated HindIII linkers. Following digestion with HindIII and Xbal, 5' truncated CD68 promoter fragments were gel purified and subcloned into HindIII and Xbal digested pCATBasic to give the plasmids - 2258CD68pCAT, -1576CD68pCAT and -951CD68pCAT. All other CD68 promoter deletions were prepared by PCR using plasmid -2940 CD68pCAT as a template and 5' oligonucleotide primers which added a HindIII site and a common 3' PCR primer which spanned the Xbal cloning site of plasmid -2940 CD68pCAT (listed in Table 1). Amplified fragments were digested with HindIII and Xbal, gel purifed and subcloned into HindIII and Xbal digested pCATBasic to give the plasmids -333CD68pCAT, - 232CD68pCAT, -150CD68pCAT and -80CD68pCAT. The first intron of the CD68 gene was PCR amplified using primers 5' ccggaattcTGCTGGGGCTACTGGCAG and 5' tgatctagaGTCCCCTGGGCTTTTGGCAG which added EcoRI and Xbal sites (underlined). Following EcoRI and Xbal digestion the CD68 intron fragment was cloned into pCD68Bst3-2 digested with EcoRI and Xbal to give plasmid pCD68BstIVS and a 3022bp Hindlll - Xbal fragment was cloned into pCATBasic to give the plasmid -2940 IVSpCAT. The HIV minimal LTR construct HIV pCAT is from Lew et al. (1991) Mol. Cell. Biol. 11, 182-191. Construct HIV IVS pCAT was made by ligating the EcoRI to Xba I IVS I fragment of pCD68BstIVS into the unique BgIII site in the HIV tar sequence. All CD68 promoter reporter constructs were sequenced using M13 reverse and CAT primers and shown to exactly match the CD68 promoter sequence shown in Figure.A 1.7 kb HindIII-BamHI (blunt) fragment containing CD11b sequences from -1706 to +91 was excised from the plasmid pB202 (Dzjemmis *et al*, 1995, Blood, 85, 319-329 and cloned between the HindIII and Xbal (blunt) sites of pCATBasic to give the clone CD11b pCAT. A 516bp Hind III - Xba I fragment containing c-fes sequences from -446 to +71 was excised from the plasmid p446 (a kind gift of Celeste Simon, Heydemann et al., 1996) and cloned between the Hind III and Xba I sites of pCATBasic to give the plasmid c-fes pCAT. Other myeloid gene promoters were cloned by PCR using the primers designed from published sequences listed in Table I with cloned DNA or human genomic DNA templates. A Sma-BamHI fragment containing the SV40 early splice and polyadenylation sequences from plasmid pMSG (Pharmacia) was ligated into Smal-BamHI digested plasmid pH2KBS which contains the 1.6kb H2K cDNA cloned into the EcoRV site of pBluescript (a kind gift of Dr D. Mokophidis) to give the plasmid pH2KSV. A 2.5kb HindIII-BamHI fragment of pH2KSV was rendered blunt ended by treatment with T4 DNA polymerase and ligated into Smal digested CD68 promoter plasmid pCD68Bst3-2 to give plasmid pCD68-H2KSV and the same pH2KSV fragment was ligated into Hincll digested human lysozyme promoter plasmid pBH7.4 (Clarke et al. 1996) to give hLZM-H2KSV. Plasmid pkb-HindIII contains a 7.4kb HindIII genomic DNA fragment of the H2Kb gene (Weiss et al., 1992).

Supercoiled plasmid DNAs were prepared from 500ml cultures of E.coli by NaOH/SDS lysis and purified by equilibrium centrifugation in CsCl/ethidium bromide gradients followed by phenol/chloroform extraction and ethanol precipitation (Sambrook, Fritsch & Maniatis 1989).

### Mammalian cell culture and transient transfection

The murine macrophage cell lines RAW264.7 and P388.D1 were maintained in RPMI 1640 medium (Gibco BRL) supplemented with 10% heat inactivated foetal calf serum (FCS) (Sigma), 100 units ml-1 penicillin, 100 µg ml-1 streptomycin, 2mM glutamine and 10mM Hepes (pH 7.0). CHO K1 cells were maintained in Ham's F-12 medium (Gibco BRL) supplemented with 10% FCS, antibiotics and glutamine. All cells were grown at 37oC in a humidifed incubator in 5% CO2/air. RAW264.7 and P388.D1 cells were grown to confluence in T175 flasks, harvested in Phosphate Buffered Saline (PBS) and washed once and resuspended in Optimem 1 serum free medium (Gibco BRL) for RAW cells or RPMI 1640 (no FCS) for P388D1 cells. Cells were counted and adjusted to a final density of 4 x 107 cells ml-1. Aliquots of 2 x 107cells (0.5ml) were mixed with 50 µg CAT reporter plasmid DNA and 5 µg pcDNA3 b-galactosidase plasmid DNA, added to a 0.4cm electrode gap electroporation cuvette (BioRad) and shocked in a BioRad GenePulser (300V, 960 µFD) at room temperature. Cells were recovered immediately into 10ml of cell growth medium which had been pre-warmed to 37°C and plated into 35mm and 9cm diameter tissue culture petri dishes (Nunc). Cells were analysed 24 or 48 hours post electroporation for transfection efficiency by staining fixed permeabilised cells with 5-bromo-4-chloro-3-indolyl-β-D-galactopyranoside (X-gal, Sigma) as described (Hogan et al. 1994). Transient transfection efficiencies of between 20 and 30% were routinely obtained with P388.D1 murine macrophages and after optimisation transient transfection efficiencies in excess of 40% could be obtained with RAW264.7 cells. CHO cells were grown to 70-80% confluence in 9cm petri dishes, washed twice with Optimem before addition of 5ml of plasmid DNA:cationic lipid complex (5 µg DNA:50 µg Lipofectamine (Gibco BRL) in Optimem). After 6-16 hours incubation the medium was aspirated, cells were washed twice with PBS and recovered into complete medium for 24-36 hours before analysis. X-gal staining and FACS analysis routinely showed CHO cell transient transfection efficiencies in excess of 40%.

### Reporter gene assays

Transfected cells were harvested by scraping in PBS, washed once with PBS and cell pellets were resuspended in 100µl 0.25M Tris-HCl (pH 7.8) and subjected to three rounds of freeze thaw lysis. Cell lysates were assayed for β-galactosidase enzyme activity using the colorimetric substrate chlorphenolred β-D-galactopyranoside (CPRG, Boehringer Mannheim) in a 96 well plate assay in 50mM potassium phosphate buffer (pH7.3) with 2mM MgCl2. Enzyme activity was determined by spectrophotometry at 570nm after 30 minutes incubation at 37oC using dilutions of purified E.coli β-galactosidase enzyme (Sigma) to generate a standard curve. CAT enzyme activity of cell lysates was determined after heat treatment (65oC, 20 minutes) using 10 µCi 14C labeled chloramphenicol (54Ci mmol-1, Amersham) as substrate in a 125µl reaction in 0.25M Tris-HCl (pH 8.0) containing 0.2 mg ml-1 n-butyryl CoA as cofactor. CAT enzyme activity was measured by determining the amount of butyryl-14C chloramphenicol extracted into mixed xylenes (Aldrich) after a 2 hour incubation at 37oC (Seed ref). A CAT enzyme standard curve was generated using dilutions of purified CAT enzyme (Promega) in each experiment and all CAT enzyme assays using transfected cell extracts were within the linear range of the enzyme assay.

### Figure 1. Restriction maps of cosmids containing the human CD68 gene.

Recombinant cosmids containing the human CD68 gene in the vector pWE15 were isolated by PCR screening of pools of robotically picked Ampicillin resistant HB101 colonies using PCR primers CD68 L1 & CD68 L2. CD68 PCR positive single colonies were used to prepare cosmid DNA which was analysed by restriction mapping and Southern blotting using radioactively labelled CD68 gene probes. Sites for the restriction enzymes Spe I (Spe), Cla I and Not I are shown. Not I sites in brackets (Not I) are derived from the pWE15 cosmid vector cloning site and flank the human genomic DNA insert.

Below the map of cosmid cosCD68C1 is shown the position of a 20kb EcoRI fragment cloned into the plasmid vector pBluescriptSK to give the recombinant plasmid pCD68R1A. The positions of other cosCD68C1 restriction fragments cloned into the plasmid vector pBluescriptSK- are also indicated. The 3kb BstXI fragment the 3' end of which contains the CD68 gene ATG initiation codon is indicated.

### Figure 2. DNA sequence of the 5' flanking region of the human CD68 gene.

The DNA sequence of the 5' flanking region of the CD68 gene was determined by double strand sequencing using the CD68 cosmids as template and oligonucleotide primers derived from the published human CD68 cDNA sequence (17).The initiator Methionine encoding ATG codon of the CD68 gene which is contained within a BstXI restriction enzyme recognition site is boxed. CD68 coding regions are underlined and intervening sequences inferred from comparison with the published CD68 cDNA sequence are delimited by vertical arrows. The sequence has not yet been confirmed on both strands, ambiguities in multiple sequence reads are indicated by lowercase letters according to the IUPAC-IUB standards described in Nuc. Acids. Res. 13, 3021-3030 (1985). Dots in the sequence indicate a chemical bond.

### Figure 3. Northern blot analysis of RNA expression in stably transfected RAW cells.

Total RNA was prepared from G418 resistant RAW cell populations (derived from at least 10,000 independent G418 resistant colonies) or polyclones (derived from 50 -100 independent G418 resistant colonies). RAW cells were transfected with 10 µg of supercoiled or Pvu I linearised CD68 cosmids or a pcDNA3 CAT plasmid which confers resistance to G418.

Total RNA (10 µg) was denatured with formaldehyde, subjected to agarose gel electrophoresis, transferred to nylon membranes and hybridised with radioactively labelled human CD68 and mouse lysozyme probes. For comparison total RNA (5 µg) prepared from human THP1 cells treated with PMA for 24 hours and human PBMC cultures was analysed. A 6 hour autoradiographic exposure is shown.

### Figure 4. RT PCR analysis of CD68 and macrosialin RNAs in stably transfected RAW cells.

RNAs (10 µg) prepared from the G418 resistant RAW cell populations analysed by Northern blotting in Figure 3 were used to prepare oligo dT-primed cDNA in a reverse transcription (RT) reaction in a final volume of 100 µl. Control RT reactions omitting reverse transcriptase were performed using the same RNA samples (-RT). A 30 cycle Polymerase Chain Reaction (PCR) using macrosialin and CD68- specific primers was performed using 1 µl of the neat RT reaction or indicated dilutions as template. The RT PCR products were analysed by agarose gel electrophoresis and the position of mouse macrosialin and human CD68 PCR products of the predicted sizes are indicated.

### Figure 5. Northern blot analysis of RNA expression in stably transfected RAW and A20 cells.

RAW and A20 B-cells were transfected with 10 µg of supercoiled or Pvu I linearised CD68 cosmids or a pcDNA3 CAT plasmid which confers resistance to G418. Total RNA (10 µg) prepared from G418 resistant cell populations was denatured with formaldehyde, subjected to agarose gel electrophoresis, transferred to nylon membranes and hybridised with a radioactively labelled human CD68 probe. For comparison total RNA prepared from human THP1 cells treated with PMA for 24 hours (5 µg) and human PBMC cultures (2 µg) was analysed. A 6 hour autoradiographic exposure is shown.

### Figure 6. RT PCR analysis of CD68 and HPRT RNAs in stably transfected RAW and A20 cells.

RNAs (10 µg) prepared from the G418 resistant RAW cell populations analysed by Northern blotting in Figures 3 & 5 were used to prepare oligo dT-primed cDNA in a reverse transcription (RT) reaction in a final volume of 100 µl. A 30 cycle Polymerase Chain Reaction (PCR) CD68- and HPRT- specific primers was performed using 1R1 of the neat RT reaction or indicated dilutions as template. The RT PCR products were analysed by agarose gel electrophoresis and the position of human CD68 PCR and HPRT PCR products of the predicted sizes are indicated.

**Table 1 This table shows the effect of deleting CD68 5' promoter seq uences in RAW and P388.D1 transfections**

| | | |
|---|---|---|
| -2940pCAT | 38.6 | 78.4 |
| -666pCAT | 37.5 | 74.4 |
| -575pCAT | 48.4 | 129.5 |
| -460pCAT | 71.6 | 132.2 |
| -333pCCAT | 30.5 | 112.3 |
| -232pCAT | 34.4 | 96.4 |
| -150pCAT | 11.6 | 176 |
| -80pCAT | 0.64 | 9.5 |
| pCATBasic | 0 | 2 |
| pCATControl | 100 | 100 |
| Plasmid construct | RAW264.7 | P388.D1 |

**Table 2 This table compares the level of CAT reporter gene activity of plasmids with different myeloid gene promoters in P388.D1 and RAW264.7**

| | | |
|---|---|---|
| pCAT Control | 100 | 100 |

| | | |
|---|---|---|
| pCAT Basic | 0 | 8.4 |
| -2940 CD681VS | 117 | 163.9 |
| -2940 CD68 pCAT | 39 | 50 |
| hLZM pCAT | 17.6 | 66 |
| mLZM pCAT | 20.1 | 73.3 |
| CD11b pCAT | 42 | 23 |
| -c-fes pCAT | 4.8 | 47.3 |
| hMSR pCAT | 3 | 35.5 |
| CAT Reporter | RAW264.7 | P388.D1 |
| Plasmid | | |

**Table 4 This table compares the level of CAT reporter gene activity of plasmids with CD68 and HIV minimal LTR promoters in P388.D1 and RAW264.7 cells**

| | | |
|---|---|---|
| pCAT Control | 100 | 100 |
| pCAT Basic | 0 | 8.4 |
| -2940 CD681VS | 117 | 163.9 |
| -2940 CD68pCAT | 39 | 50 |
| HIV IVS pCAT | 0.8 | 15 |
| HIVpCAT | 0.8 | 69 |
| CAT Reporter | RAW264.7 | P388D1 |
| Plasmid | | |

**Table 5 This table shows the effect of adding the CD68 IVS intron on to different CD68 promoter fragments in RAW and CHO cells**

| | | |
|---|---|---|
| pCAT Control | 100 | 100 |
| pCAT Basic | 0 | 0 |
| -2940 CD68pCAT | 31 | 56 |
| -2940 IVS | 154 | 63 |
| -80 pCAT | 0.6 | 5 |
| -80 IVS | 53 | 1.9 |
| Plasmid construct | RAW264.7 | CHO |

### Potential Applications for the CD68 LCR

### The Macrophage as a delivery vehicle for gene therapy

Macrophages have several important advantages over other cell types for delivering therapeutic gene products in a range of important human diseases

Macrophages have a high biosynthetic capacity. Macrophages secrete physiologically significant amounts of cytokines, growth factors, inflammatory mediators, proteases, protease inhibitors and other important biologically active macromolecules.

Macrophages have a limited life span. After commitment tissue resident and recruited macrophages undergo only one or two cell divisions at most, this would be a distinct advantage in many human gene therapy protocols.

Macrophages are found in virtually all tissues. The presence of macrophages in virtually every tissue in the body in significant numbers increases the utility of an LCR which directs macrophage-specific gene expression. The presence of macrophages in the lung and gut allows for recombinant DNA delivery to macrophages by a number of different routes.

Macrophages are rapidly recruited into sites of inflammation. The ability to direct heterologous gene expression in a cell type which is recruited to sites of inflammation offers unique avenue for therapeutic intervention in chronic inflammatory disease.

### Somatic Gene Therapy

There are currently no human monogenic disorders described which specifically affect macrophage function which would be candidates for "classical" somatic gene therapy. However the CD68 LCR could be used to drive the expression of therapeutic gene products in human macrophages in a whole range of important human diseases. Candidate genes would indude: Local delivery of soluble IL-1Ra to inflammed rat knee joints by recombinant retroviral vectors has been shown to be 10 000 times more efficient than systemic administration of sIL-1 RA in reducing experimentally-induced arthritis (9) Makarov, S.S., *et al* Proc. Natl. Acad. Sci. (USA), 1996, **93**; 402-406.

Currently there is much interest in anti **TNF**-α therapy in treatment of toxic shock syndrome and a number of autoimmune diseases such as rheumatoid arthritis which afflicts 1% of the UK population. Many important human diseases are the result of a failure in regulation of the immune system. Macrophages and cytokines secreted by macrophages such as IL-12 and IL-10 play a key role in regulating T-lymphocyte function. Macrophages-specific expression of transdominant negative IL-4 receptors could find application in autoimmune diseases such as Ulcerative Colitis and Crohn's Disease.

Apo E and several other gene products have been proposed to play a key role in the development of atherosclerotic plaques which are the cause of blood vessel occlusion in atherosclerosis and strokes. Apo E expressed in macrophages present in atherosclerotic plaques might find application in the treatment of vascular occlusion (10). CD68 is present in the macrophage derived foam cells found in human disease tissue and we have shown the mouse homologue of CD68, macrosialin, to be present at high levels in the atherosclerotic plaques of an apo E^{-/-} mouse model of atherosclerosis.

The murine homologue of CD68, macrosialin has been reported to bind oxidised LDL (11). Overexpression of **human CD68, murine macrosialin or human Macrophage Scavenger Receptor** in macrophages present in atherosclerotic plaques may usefully reduce the amount of atherogenic oxidised LDL in atherosclerotic lesions.

Chronic Granulomatous Disease (CGD) is a genetic disease caused by mutation of the NAPDH oxidase gene. CGD patients fail to make reactive oxygen metabolites in their phagocytic cells which kill bacteria and hence patients suffer from recurrent bacterial infections. Expression of NAPDH oxidase in macrophages of CGD patients by somatic gene therapy would be highly beneficial (12).

There are several relatively rare human genetic diseases which can be treated by providing purified proteins which can be taken up by defective cells to correct their genetic defect. Examples include **Beta cerebrosidase** in patients with Gaucher's disease and other genes defective in lysosomal storage disorders (13). A particular attraction of the CD68 LCR for treatment of lysosomal storage diseases is the fact that CD68 is expressed in microglia, mononuclear phagocytic cells resident in the brain. Some microglial cells in adults are derived from recruited blood monocytes and hence the CD68 LCR may offer the possibility of expressing therapeutic gene products in the brain via recruited blood monocytes transfected with CD68 LCR vectors.

### Treatment of infectious diseases

Several important human pathogens survive and replicate in the endosomal compartment of macrophages. These include the causative agents of tuberculosis, leismaniasis and leprosy. The HIV virus survives and replicates in monocytes. Targeting γ-interferon production to macrophages infected with Mycobacterium bovis could be a viable strategy for treatment of Tb. The delivery of HIV decoy tar sequences and other anti HIV reagents to monocytes and macrophages would create a pool of monocytes resistant to HIV infection. Similar gene therapy protocols for the "intracellular vaccination" of T-lymphocytes have been proposed.

### Macrophage Expression Systems

The original β- globin LCR vectors have been used to develop expression systems for the over production of heterologous gene products in cultured erythroid cell lines(14). The CD68 LCR could be used for over expression of heterologous genes in human and murine macrophage cell lines for instance in the production of cytokines and soluble receptors whose pattern of glycosylation was important for their biological activity.

### Genetic Vaccination

Recently it was shown that naked DNA could be used in animals to confer protective immunity against lethal virus challenge and to elicit significant humoral antibody responses. Current protocols for genetic vaccination use standard mammalian expression vectors based on the SV40 or HCMV promoters and enhancers. Heterologous genes are expressed in myofibres but the cells presenting foreign antigen for the elaboration of an effective immune response are unknown (15). Using a CD68 LCR vector to target heterologous gene expression to macrophages and possibly dendritic cells should increase the efficiency and efficacy of genetic vaccination protocols.

### Immunotherapy

Dendritic cells process antigens for presentation to cells of the immune system. Human dendritic cells expressing the CD68 antigen are important in conferring tolerance in organ transplant rejection and autoimmune diseases. A CD68 LCR will allow for the genetic targeting of an important subset of Dendritic cells grown in culture from bone marrow or peripheral blood precursors.

### Compositions

The invention also relates to compositions comprising the polynucleotide, vector or transfected cell. Thus, the polypeptides of the present invention may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of a polypeptide of the invention and a pharmaceutically acceptable carrier or excipient. Such carriers may include, but are not limited to, saline, buffered saline, dextrose, water, glycerol, ethanol and combinations thereof. The formulation should suit the mode of administration.

### Kits

The invention further relates to diagnostic and pharmaceutical packs and kits comprising one or more containers filled with one or more of the ingredients of the aforementioned compositions of the invention. Associated with such container(s) can be a notice in the form prescribed by a govemmental agency regulating the manufacture, use or sale of pharmaceuticals or biological products, reflecting approval by the agency of the manufacture, use or sale of the product for human administration.

### Administration

Polynucleotides and other compounds of the present invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

The pharmaceutical compositions generally are administered in an amount effective for treatment or prophylaxis of a specific indication or indications. In general, the compositions are administered in an amount of at least about 10 µg/kg body weight In most cases they will be administered in an amount not in excess of about 8 mg/kg body weight per day. Preferably, in most cases, dose is from about 10 µg/kg to about 1 mg/kg body weight, daily. It will be appreciated that optimum dosage will be determined by standard methods for each treatment modality and indication, taking into account the indication, its severity, route of administration, complicating conditions and the like.

In therapy or as a prophylactic, the active agent may be administered to an individual as an injectable composition, for example as a sterile aqueous dispersion, preferably isotonic.

Alternatively the composition may be formulated for topical application for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For administration to mammals, and particularly humans, it is expected that the daily dosage level of the active agent will be from 0.01 mg/kg to 10 mg/kg, typically around 1 mg/kg. The physician in any event will determine the actual dosage which will be most suitable for an individual and will vary with the age, weight and response of the particular individual. The above dosages are exemplary of the average case. There can, of course, be individual instances where higher or lower dosage ranges are merited, and such are within the scope of this invention.

A vaccine composition is conveniently in injectable form or may be administered by other techniques, for example a gene gun using, for example, gold particles, or *ex vivo* Conventional adjuvants may be employed to enhance the immune response.

A suitable unit dose for vaccination is 0.5-5µg/kg of antigen, and such dose is preferably administered 1-3 times and with an interval of 1-3 weeks.

With the indicated dose range, no adverse toxicological effects will be observed with the compounds of the invention which would preclude their administration to suitable individuals.
The polynucleotide sequences, vectors and host cells of the present invention may be used in the treatment of infectious diseases including viral and bacterial infections such as HIV and TB, inflammatory diseases, cardiovascular diseases, rheumatoid arthritis, atherosclerosis, restinosis, cancer, for example cancer of the bowel, colon, breast or lung.

### Other LCR Patents

The published LCR most similar in targeting transgene expression to macrophages is the Class II LCR. This element has been claimed to direct expression to (presumably activated) macrophages, dendritic cells and B-lymphocytes (16). Our data with stably transfected murine macrophage line RAW 264.7 and the murine B-cell line A20 already show the CD68 cosmids direct expression which is restricted to macrophages (Figures 5 8 6).

### References

1. Grosveld, F., Blomvan Assendelft, M., Greaves, D.R. and Kollias, G. Position-independent, high-level expression of the human β- globin gene in transgenic mice. Cell, 1987, **51**, 975- 985.
2. Greaves, D.R., Wilson, F.D., Lang, G. and Kioussis, D. Human CD2 3' flanking sequences confer high- level, T- cell specific, position independent gene expression in transgenic mice. Cell, 1989, 56, 979- 986.
3. Madisen, L. and Groudine, M. Identification of a locus control region in the immunoglobulin heavy-chain locus that deregulates c-myc expression in plasmacytoma and Burkitt's lymphoma cells. Genes and Development, 1994, 8, 2212- 2226.
4. Blom van Assendelft, M., Hanscombe, O., Grosveld, F. and Greaves, D.R.
   The β- globin dominant control region activates homologous and heterologous promoters in a tissue- specific manner. Cell, 1989, **56**, 969- 977.
5. Talbot, D., Collis, P., Antoniou, M., Vidal, M., Grosveld, F. & Greaves, D.R.
   A dominant control region from the human, β globin locus conferring integration site- independent gene expression. Nature, 1989, 338, 352- 355.
6. Therexsys and gene therapy
   Nature Medicine, 1995, **1**, 502
7. Dighe, A.S., Campbell, D., Hsieh,C-S, Clarke, S., Greaves, D.R., Gordon, S., Murphy, K.M. and Schreiber, R.D. Tissue-specific targeting of cytokine unresponsiveness in transgenic mice.
   Immunity, 1995, **3,** 657- 666.
8. Clarke, S., Greaves, D.R., Ping Chung, L, Tree, P. and Gordon,S. The human lysozyme promoter directs reporter gene expression to activated myelomonocytic cells in transgenic mice.
   Proc. Natl. Acad. Sci. (USA), 1996, **93**; 1434- 1438.
9. Makarov, S.S., *et al.*
   Suppression of experimental arthritis by gene transfer of interleukin 1 receptor antagonist cDNA
   Proc. Natl. Acad. Sci. (USA), 1996, **93**; 402- 406.
10. Linton, M.F., Atkinson, J.B. and Fazio, S.
   Prevention of Atherosclerosis in Apolipoprotein E-deficient mice by bone marrow transplantation.
   Science (1995) 267; 1034-1037.
11. Ramprasad, M.P., Fischer, W., Witzum, J.L., Sambrano, G.R.,
   Quehenberger, O. and Steinberg, D.
   The 94-97-kDa mouse macrophage membrane protein that recognises oxidized low density lipoprotein and phosphatidylserine-rich liposomes is identical to macrosialin, the mouse homologue of human CD68.
   Proc. Natl. Acad. Sci. (USA), 1995), **92**; 9580-9584.
12. Segal, A.W.
   The NAPDH oxidase and chronic granulomatous disease. Molecular Medicine Today, 1996, **2**; 129- 135.
13. Zhou, X.Y., Morreau, H., Rottier, R, Davis, D., Bonten, E., Gillemans, N., Wenger, D., Grosveld, F.G., Doherty, P., Suzuki, K., Grosveld, G.C. and d'Azzo, A.
   Mouse model for the lysosomal disorder galactosialidosis and correction of the phenotype with overexpressing erythroid precursor cells.
   Genes and Development (1995) 9; 2623- 2634.
14. Needham, M. *et al.*
   LCR/MEL: A versatile system for high-level expression of heterologous proteins in erythroid cells.
   Nucleic Acids Research, 1992, **20**; 997-1003.
15. Maurice, J.
   Gene vaccines
   Molecular Medicine Today, 1995,1; 64-71.
16. Carson, S. and Wiles, M.
   Far upstream regions of class 11 MHC Ea are necessary for position-independent, copy-dependent expression of an Ea transgene.
   Nucleic Acids Research, 1993, 21; 2065- 2072.
17. Holness, C. and Simmons, D.
   Molecular cloning of CD68, a human macrophage marker related to iysosomal glycoproteins.
   Blood, 1993, **81**;1607-1613.

Seq ID No. 1
REVERSE-COMPLEMENT of: Drg 237.Con check: 1236 from: 1 to: 3601

Drg237.rev Length: 3601 April 29, 1996 11.13 Type N Check: 6126

1 TGTCCTGGAA CCCAGGTGCC TACCTGGTCT GCTGCATATT TGTTTTCTCT
51 TCCAGCATGG AGATATGGnA CCAAAAGGAA CGAGTGCTCA GAGTTTTGAT
101 TACCAnTGAC CTGCTGGTGA GTAGAGGGAA CTGATAGCAA AGGCAGAAGG
151 GAGGATCCAA GGTGATTCCC TCTCCAAGGC AAGTTCGGAA AGTAGCAGCT
201 TGGAATAGAA TCTGGCATGC CTAAGGCCTT TGGGGAACTG GGATGCTTAT
251 TTCCTCTGCC TTCCTTGGCT GCCCACATGG ATGCCTAAGT GTCTTCCCTC
301 CGGGATAGAG TGTCCTCCGT GCACATGCTG AAGAGTTGTC TTTCTTGACG
351 TAGGCCAGAG GCATTGATGT GCAGCAGGTT TCTTTAGTCA TCAAcTATGA
401 CcTTCCCACC AACAGGGAAA ACTATATCCA CAGGTAAGCG TAGATCTGGA
451 ACATTCCCAn ACCCTTTCAC ACcTGGCCcT CCCTGGGCTT AAAGCTCCTG
501 ATATTCCTCA TCCCCTTCCT TGTTTTCCAG AATCGGTCGA GGTGGACGGT
551 TTGGCCGTAA AGGTGTGGCT ATTAACATGG TGACAGAAGA AGACAAGAGG
601 AyTcTTCGAG ACATTGAGAC cTTCTACAAC ACCTCCATTG AGGAAATGCC
651 CCTCAATGTT GCTGACCTCA TCTGAGGGGC TGTCcTGCCA CCCAsCCCCA
701 gCCAsgGcTC AAkyTcTGGG GGCTGAGGAk CwgCAGGAGG GGGGAGGGAA
751 GGGAGCCAAG GGATGGACAT CTTGTcAtTT TTTTTtCTTT GAATAAATGT
801 CACTTTTTGA GGCAAAAGAA GGAACCGTGA ACATTTTAGA CACCCTTTTC
851 TTTGGGGTAG GCTCTTGCCC CAGGCGCCGG CTCTTCTCCC AAAAAAAAAA
901 AAAAAACAcT AaTCCATTTC CCTAACcTAg TAACcTCCAG ATCCCAGAGG
951 CTCTCCTCAC CTCAGCTGAG CTCCTTTGAA AGTGATTCAA GGGAcTATGT
1001 CAcTCAgCcT CATTTGcTGG ACCAAATcTG GAGGGAGAAC CCCTAAAACC
1051 CcTAAGTGAG GTTGCCCAGG GGGTTGTCCC CAGGTGGGGG GAAGCAGGGG
1101 AGAGAAAATG GTAGCCATTT TtACATTGTT TTGTATAGTA TTTATTGATT
1151 CAGGAAACAA ACACAAAATT cTGAATAAAA TGACTTGGAA ACTGCCTGTT
1201 TGGGCTTCTC ATTTCTtACC TCCCCTTCCC TCTCCCACCT GmTAcTGGGT

1251 GCATcTCTGC T.CCCCCCTT .CCCCAGCAG ATGGTTACCT TTGGGCTGTT
1301 GCTTTCTTGT CACCATCTGA GTTCTCAGAC GCTGGAAAGC CATGTTCTCG
1351 GCTCTGTGAA tGACAATGcT GAcTGGAGTG CTGCCCCTCT GTAAAGGGcT
1401 GGGTGTGGAT GGTCACAAGC CCtTcACATG CyTCAGCCAA GAgGAAGTAG
1451 tACAGGggTC AGCCCAgAgg TCCAGGGGAA AgGAgtgGAA AcCGATTTCC
1501 CCACCAA.GG GAgGGGCcTG TACcTCAgcT GTTCCCATAg cTACTTGCCA
1551 CAACTGCCAA GCAAgTTTCG cTGAgTTtGA CACATGG.AT CCC.TGTGGA
1601 TCAAcTGCCc TAgGAcTCcG TTTGCACCCA TgtgACacTG ttGAcTTTGC
1651 CCTGAcgAa. gCAgggcCAa cagtcccccta AcTTAATtAC aAaAAcTAAT
1701 GAcTAAGAgA gAgGTGGcTA gAgCTGAgGC CCCTG.AgTC AgGcTGTGGG
1751 TGGGATCaTc tCCAgTACAG GAAgtGAGAc TTTCATTT.C ctCCtTTcCA
1801 Ag.AgAgGGc TGAgGGAgCa gGGTTgAgCa ActGGTGCAg ACAgCCTAgc
1851 TGGActTTGG GTgAgGc..g gtTCAgCCAT gAgGctGGcT gTgCttTtcT
1901 cGGGGGcCCT GcTGGGGcTA cTGGCAGGTA AGGAGGAAgG AgGcTGAGGG
1951 GAGGGGG..c CCCTGGGAGG GAGCcTG.CC CTGGGTTGct AACCATcTCC
2001 T....ct.CT GCCAAAAGCC CAGGGGACAG GGAATGAC.T GTCCTCACAA
2051 AAAATCAGcT ACTTtGcTGC CATCCtTCAC GGTGACACCC ACGGtTACAG
2101 AGAGCACTGG AACAAcCAGC CACAGGACTA CCAAgAGCCA CAAAACCACC

Seq ID No 2

TTCCAAGAGAGGGCTGAGGGAGCAGGGTTGAGCAACTGG

TGCAGACAGCCTAGCTGGACTTTGGGTGAGGCGGTTCAGCCAGGAATCC

TGCTGGGGCTACTGGCAGgtaaggagCCCAG

gaaggaggctgaggggagggggcccctgggagggagcctgccctggg

ttgctaaccatctcctctctgccaaaagCCCAG

Seq ID No. 3

aattcggttctccaatcccctgggtcactttgctcttgtgcacgctttcc

agtctttcagcgtaagccagagtcattcccaaggatgctggtttctctct

gggggaagagctgctctgtgatggagcccatgcgtgtcatctgagcctct

ggcttccctgccagtgcagccctggcagtgtcctacttcccagggctgtt

gtctgcctggcgggaaggtcctgggcaaaggatcagtctttgtactctga

gagcagactacttggctcctctctgtttttatcagcgaagttggatata

tctctcccacatttccctaatcatatgctatatattggcttttttttct

tctctagCCCCCAAATACATCAAGATGTTTGTACTGGATGAAGCTGACGA

AATGTTAAGCCGTGGATTCAAGGACCAGATCTATGACATATTCCAAAAGC

TCAACAGCAACACCCAGgtgagggcagtcttgcttgaatagctaatgatt

cttgaaaaatagtaagtgccaggggaaaccaaatactggattcttgagcc

ttttatgcatctgcttcagttttaggtgtggctagggaagggagcaggc

ctcaggaaggaaccagcactctaagactggcctttttttccactagGTAG

TTTTGCTGTCAGCCACAATGCCTTCTGATGTGCTTGAGGTGACCAAGAAG

TTCATGAGGGACCCCATTCGGATTCTTGTCAAGAAGGAAGAGTTGACCCT

GGAGGGTATCCGCCAGTTCTACATCAACGTGGAACGAGAGgtggggccca

gtgcaggaggcgggcctggtagtgagttgttgggtatagcccctgactga

tttttgtcccccaacctccagGAGTGGAAGCTGGACACACTATGTGACTT

GTATGAAACCCTGACCATCACCCAGGCAGTCATCTTCATCAACACCCGGA

GGAAGGTGGACTGGCTCACCGAGAAGATGCATGCTCGAGATTTCACTGTA

TCCGCCATGgtgtgtttgcccgctgccagcctgttgtgggtctgcccgtc

agaagtgtcctacttgaagccagggttcctggaacccaggtgcctacctg
gtctgctgcatatttgtttctcttccagCATGGAGATATGGACCAAAAG
GAACGAGACGTGATTATGAGGGAGTTTCGTTCTGGCTCTAGCAGAGTTTT
GATTACCACTGACCTGCTGgtgagtagagggaactgatagcaaaggcaga
agggaggatccaaggtgattccctctccaaggggacatcagtgcctctca
ggaaagtagcagcttggaatagaatctggcatgcctaaggcctttgggga
actgggatgcttatttcctctgccttccttggctgcccacatggatgcct
aagtgtcttccctccgggatagagtgtcctccgtgcacatgctgaagagt
tgtctttcttgacgtagGCCAGAGGCATTGATGTGCAGCAGGTTTCTTTA
GTCATCAACTATGACCTTCCCACCAACAGGGAAAACTATATCCACAGgta
agcgtagatctggaacaytcccntacccnttcacacctggccctccctgg
gcttaaagctcctgatattcctcatccccttccttgttttccagAATCGG
TCGAGGTGGACGGTTTGGCCGTAAAGGTGTGGCTATTAACATGGTGACAG
AAGAAGACAAGAGGANTCTTCGAGACATTGAGACCTTCTACAACACCTCC
ATTGAGGAAATGCCCCTCAATGTTGCTGACCTCATCTGAGGGGCTGTCCT
GCCACCCASCCCCAGCCAGGGCTCAAAGTCTGGGGGCTGAGGACCTGCAG
GAGGGGGGAGGGAAGGGAGCCAAGGGATGGACATCTTGTCATTTTTTTTT
CTTTGAATAAATGTCACTTTTTGAGGCAAAAGAAGGAACCGTGAACATTT
TAGACACCCTTTTCTTTGGGGTAGGCTCTTGCCCCAGGCGCCGGCTCTTC
TCCCAAAAAAAAAAAAAAACACTAATCCATTTCCCTAACCTAGTAACCT
CCAGATCCCAGAGGCTCTCCTCACCTCAGCTGAGCTCCTTTGAAAGTGAT
TCAAGGACTATGTCACTCAGCCTCATTTGCTGGACCAAATCTGGAGGGA
GAACCCCTAAAACCCCTAAGTGAGGTTGCCCAGGGGGTTGTCCCCAGGTG

GGGGGAAGCAGGGGAGAGAAAATGGTAGCCATTTTTACATTGTTTTGTAT

AGTATTTATTGATTCAGGAAACAAACACAAAATTCTGAATAAAATGACTT

GGAAACTGCCTGTTTGGGCTTCTCATTTCTTACCTCCCCTTCCCTCTCCC

ACCTGCTACTGGGTGCATCTCTGCTCCCCCCTTCCCCAGCAGATGGTT

ACCTTTGGGCTGTTGCTTTCTTGTCACCATCTGAGTTCTCAGACGCTGGA

AAGCCATGTTCTCGGCTCTGTGAATGACAATGCTGACTGGAGTGCTGCC

CCTCTGTAAAGGGCTGGGTGTGGATGGTCACAAGCCCCTCACATGCCTCA

GCCAAGAGGAAGTAGTACAGGGGTCAGCCCAGAGGTCCAGGGAAAGGAG

TGGAAACCGATTTCCCCACCAAGGGAGGGGCCTGTACCTCAGCTGTTCC

CATAGCTACTTGCCACAACTGCCAAGCAAGTTTCGCTGAGTTTGACACAT

GGATCCCTGTGGATCAACTGCCCTAGGACTCCGTTTGCACCCATGTGA

CACTGTTGACTTTGCCCTGACGAA.GCAGGGCCAACAGTCCCCTAACTTA

ATTACAAAAACTAATGACTAAGAGAGAGGTGGCTAGAGCTGAGGCCCCTG

AGTCAGGCTGTGGGTGGGATCATCTCCAGTACAGGAAGTGAGACTTTCA

TTTCCTCCTTTCCAAGAGAGGGCTGAGGGAGCAGGGTTGAGCAACTGG

TGCAGACAGCCTAGCTGGACTTTGGGTGAGGCGGTTCAGCCATGAGGC

TGGCTGTGCTTTTCTCGGGGGCCCTGCTGGGGCTACTGGCAGgtaaggag

gaaggaggctgaggggagggggcccctgggagggagcctgccctggg

ttgctaaccatctcctctctgccaaaagCCCAGGGGACAGGGAAT

GACTGTCCTCACAAAAAATCAGCTACTTTGCTGCCATCCTTCACGGTGA

CACCCACGGTTACAGAGAGCACTGGAACAACCAGCCACAGGACTACCAAG

AGCCACAAAACCACCACTCACAGGACAACCACCACAGGCACCACCAGC

CACGGACCCACGACTGCCACTCACAACCCCACCACCACCAGCCATGGA
AACGTCACAGTTCATCCAACAAGCAATAGCACTGCCACCAGCCAGGGACC
CTCAACTGCCACTCACAGTCCTGCCACCACTAGTCATGGAAATGCCACGG
TTCATCCAACAAGCAACAGCACTGCCACCAGCCCAGGATTCACCAGTTCT
GCCCACCCAGAACCACCTCCACCCTCTCCGAGTCCTAGCCCAACCTCCA
AGGAGACCATTGGAGACTACACGTGGACCAATGGTTCCCAGCCCTGTGT
CCACCTCCAAGCCCAGATTCAGATTCGAGTCATGTACACAACCCAGGGTG
GAGGAGAGgtaaagctaaaactgggggatgagaggggagggaggcaggac
tggttataggctcagagggaagaaggaagaggggacaggnaaccttggcc
ggcatcgcatgcagtcttgtgaccttccagtcttaacttccgcagGGCT
GGGGTATCTCTGTNCTGANCCCCAACAGAACCAAGGTCCAGGGAAGCTGT
GGGGGTGCCCATCCCCACCTGCTTCTCTCATTCCCCTATGGACACCTCAG
CTTTGGATTCATGCAGgtatagccatgacctcagtctcacccctcactca
gcctcccggcgcccctcccctcccaatcccacacgctactccttcctctg
tggagagggataccacctgcgccttcctcttcgccccacagGACCTCC
AGCAGAAGGTTGTCTACCTGAGCTACATGGCGGTGGAGTACAATGTGTC
CTTCCCCCACGCAGCACgtaagtaacctccttccctttctcattgctacc
actagacgccagggttcctgaaaggactaagctggggccagggaggtgg
ataggatctgacccttcctcactcctccagAGTGGACATTCTCGGCTCAG
AATGCATCCCTTCGAGATCTCCAAGCACCCCTGGGGCAGAGCTTCAGTTG
CAGCAACTCGAGCATCATTCTTTCACCAGCTGTCCACCTCGACCTGCTCT
CCCTGAGGCTCCAGGCTGCTCAGCTGCCCCACACAGGGGTCTTTGGGCAA
Agtaagacctacctactccttccctcctagaatcctcccactgcactgaa

aacccccttccccaggcccataagccactcatctctcttcttaaccccccca
aatctcgctctcccagcttgtcatggctacagggcagctttctttccatc
ctctacaagactctgccagtttccccctttatcactgctgagtcactgc
ggtgagctcctcaccaatctcctactccccagcatccccccattccctcc
tcccacctttatcccaaccagcacgtcactgcaaatacctacctgcccta
tccttccgccagGTTTCTCCTGCCCCAGTGACCGGTCCATCTTGCTGCCT
CTCATCATCGGCCTGATCCTTCTTGGCCTCCTCGCCCTGGTGCTTATTGC
TTTCTGCATCATCCGGAGACGCCCATCCGCCTACCAGGCCCTCTGAGCAT
TTGCTTCAAACCCCAGGGCACTGAGGGGGTTGGGGTGTGGTGGGGGGGT
ACCCTTATTTCCTCGACACGCAACTGGCTCAAAGACAATGTTATTTTCCT
TCCCTTTCTTGAAGAACAAAAAGAAAGCCGGGCATGACGGCTCATGCCTG
TAATCCCAGCACTTTGGGAGGCTGAGGCAGGTGGATCACTGGAGGTCAGG
AGTTTGAGACCAGCCTGGCCAACATGGTGAAACCCTGTCTCTACTAAAAA
TACAATTAGCCAGGTGTGGCGGCGTAATCCCAGCTGGCCTGTAATCCCAG
CTACTTGGGAGGCTGAGGCAGAACTGCTTGAACCCAGGAGGTGGAGGTTG
CAGTGAGCCGTCATCGCGCCACTGAGCCAAGAGTCGCGCCACTGCACTCC
AGCCTGGGCGACAGAGCCAGACTGTCTCAAATAAATAAATATGAGATAAT
GCAGTCGGGAGAAGGAGGGAGAGAATTTTATTAAATGTGACGAACTGCC
CCCCCCCCCCCCCCCCAGCAGGAGAGCAGCAAAATTTATGCAAATCTTTG
ACGGGGTTTTCCTTGTCCTGCCAGGATTAAAAGCCATGAGTTTCTTGTCA
CATGCCTTTCTATGCCTTCCATGGCTGGGTCTCAGGGAGCCGGAAGCAGC
TGCTGAGGAGGGATGAAAATGTCAGTGTGTGACGATGCCTCATGGGTTCA

CCCCCCAAAGCCTGGCACAGCTGGTGTTGGGTCTGCCGTGCCTCCCTTCC
TTCCTCCTCTTGGGGCCACTGGCTGCTCCAGTTCCCCATCCGTGGCAAGC
CGGTAGAGCCATTCATCCCCGCAGCCTTCTTCCTGACCCTCGTACAGTTT
CAAATGCAGCAGACAGCCAAAGCAATGAGTGGGGGCTGTGGAACTTCAT
TCCCAAAGGCAGCGCCAGTGGCTCCTGAGCAATGAGAATGTCCTGTCCTG
TCCACCATATTCAAGGCCAGCAGAAGAGCCCGATTAAACCCTCGCAGCGA
CCTGGCATGCTCCTATCCCACCTGCAAGGGGTTGAATCAAGAAGGAGCAG
TGGGTACTCTGACCTCCACTGGGGGCTCCTGGGAACAGCATGCCCCCCAC
ACGGGGCCACCTGCCAAGCCTAACTTCATGCCCCCAGTACTTGAGATGAG
GAGTGTCACTCTCAGGACAGCCAAGGTCCAGATTCTAGAAAGGACCTCCC
AGATGGCCACAGCCTGCACCAGCAGTGAGCGCCAGTCCCACCCATTACAG
CTGGCTACGGCGCAATCCCTGGGAGCCAGGATGAGCAGCACCCCCCAGCC
GTAGGAGCCCCAGGAGGCTTCCGGCTTCCAAGGCCMAGAGACTGCCCCAC
AAGGSAGCCCTCACCTGGCAGGGCCCAGCAAGCCCCACCTCTGCCTGCAG
ACATCCGTGTGACCTTGTAGACTTTGGAGGGGGCCCCAAAGGGCTGATC
CACAGCGGAATGACGCACGGGTGGGCACCGTGGGTTGGCGTCCCGGCGGT
CGGTAACGAAGCACAACGCCCCCACCAGGTAGTCCAAGGTGCCCTTTTCC
CCAAGCAGCGCTGMAGGKTCMATCTGGCCTCGCCGAAGTCTCTGCCCAGC
ACGTGGTGCCCCTCGGGCCAGCGGGAGGGGGCGAATCCCGGGACTGCTC
GCCAGGCCTCGGCTCCCCGGAGACTCTTGGGGGTSTGGGCCCCAAGGGTG
ATTCAGGTGCTGCCCTTKCCCCGACCTGGGATGCTTCCCCCACGTCTTCT
TTTGTTTTAATGTCCCGGGCCCAGCAGTTGCCGGCGCAATTCATGYTCCG
AGGCCTGAGCCAACCGGAGGCGAGACAAGCACAGGGCCCTGCGCGCAACC

CGGCACCTAAGGAGGCCTGCCCGGTGCAGACTCTCCTGCTCCCACCGGCG
CCCTTCCCTCTAGAGACGCTGAGAGAACGGAGCTAGTAGCGCCCCCACC
CAACGCCACCTCGGAGACTCCGGCTCCTTCTCTCTCAACTTCGAACAATA
CAAAGTGTGCTAGGAGAAGACAAGATGGCGCCCAGCAGGAGGAGCGGAGA
AAGGCAGGGGTGTAAATCTGGCTTCCAAACTGGAAGCGTCAACAAAGGCG
TGGGAGGTCTAACCGCGCAGGCGTGCAGCTTCGGCAAGCTT

Sequence ID No 4
ctagctggtctgagcatctctgcc ATGCGGCTCCCTGTGTGTCTGATCTTGCTAGGACCGC
TTATAG gtaaggagaaatgggaggtgggggagggagggctcatgggcaggagcctgcacctgggtggcc
aaccaatctcttactgaaag CCCAAGGAACAGAGGAAGACTGTCCTCACAAAAGGCCG
TTACTCTCCTGCCATCCTTCACGATGACACCTACAGCCACAGAAAGCACAGCCA
GCCCTACGACCAGCCACAGGCCCACCACCACCAGTCACGGAAATGTCACAGT
TCACACCAGCTCCGGACCCACAACTGTCACTCATAACCCTGCCACCACCACC
AGTCATGGGAATGCCACAATTTCTCATGCCACAGTTTCTCCCACCACAAATGGC
ACTGCTACTAGTCCAAGATCCTCCACTGTTGGCCCTCACCCTGGACCACCTCC
ACCCTCGCCTAGTCCAAGGTCCAAGGGGGCTCTTGGGAACTACACGTGGGCCA
ACGGCTCCCATCCTTGTGTTCAGCTCCAAGCCCAAATTCAAATCCGAATCCTAT
ACCCAATTCAGGGTGGAAGAAAG gtaaagctaaagtggggcttaaagagggcaagaggcaa
gtcctgggctcgttcagcagggaagaggaagagaagaggaggggataaactggatggagcattcttgtgattt
cagacccaccattgcacttctacag GCTTGGGGCATATCTGTTTTGAATCCCAACAAAACC
AAGGTCCAGGGAGGTTGTGACGGTACCCATCCCCACCTGTCTCTCTCATTTCC
TTATGGACAGCTTACCTTTGGATTCAAACAG gtatacagcttgagtttgtctctatcctctattcttcc
atatcccatacctgtacccccggagcctctgttcttgctctgtggacatggatgcctctgtccctgatgccttgagtctttyt
gttcaccttaag GACCTACATCAGAGCCCGAGTACAGTCTACCTGGACTACATGGCG
GTGGAATACAATGTGTCCTTCCCACAGGCAGCAC gtgagtaatctcttctccttaccacacta
aaagtctaggctgggcgtgctgggctggtggggaggactcaggagtcaggactggatttgactcttaattactaat
tactgcag AGTGGACATTCATGGCGCAGAATTCATCTCTTCGAGAGCTCCAAGCTC
CCTTGGGCCAAAGCTTCTGCTGTGGAAATGCAAGCATAGTTCTTTCTCCAGCTG
TTCACCTTGACCTGCTCTCTCTAAGGCTACAGGCTGCTCAGCTGCCTGACAAGG
GACACTTCGGGCCAT gtaagccctacctacttcttctttcctagagctctcccagtgctctggaaaccttccc

cagaactctttttctagacctccgcctcctctacaagactgccttatttcccctttgctgactgctctcacctatgggttggc
tcctcagcctttcttcatcccctttcctcttccgtcctcccccacctcccacctttagcccgtccaccactgcagcaattct
cagggatcccattattctgccag GTTTCTCTTGCAACCGTGACCAGTCCCTCTTGCTGCC
TCTCATCATTGGCCTGGTCCTCCTCGGCCTCCTCACCCTGGTGCTCATCGCC
TTCTGCATCACCCGCAGACGACAATCAACCTACCAGCCCCTCTGA gcatctgcccc
agtccactgtg

## Claims

1. A cloned polynucleotide having the function of a transcriptional regulatory sequence (trs) and comprising:
(a) a polynucleotide fragment having at least 70% identity to the polynucleotide of Seq ID No.2;
(b) a polynucleotide which is complementary to the polynucleotide of (a); or
(c) a polynucleotide comprising at least 15 sequential bases of the polynucleotide of (a) or (b).

2. A polynucleotide according to claim 1 wherein the polynucleotide fragment has at least 80% identity to the polynucleotide of Seq ID No. 2.

3. A polynucleotide according to claim 1 or 2 wherein the polynucleotide fragment has at least 90% identity to the polynucleotide of Seq ID No. 2.

4. A polynucleotide according to claim 1, 2 or 3 comprising the polynucleotide of Seq ID No. 2.

5. An expression cassette comprising a polynucleotide according to any one of the preceding claims and a polynucleotide operatively linked thereto encoding a heterologous polypeptide.

6. An expression vector comprising the expression cassette as claimed in claim 5.

7. A host cell comprising a vector as claimed in claim 6

8. A process for producing a polypeptide which process comprises transforming or transacting a cell with a vector as claimed in claim 6 and culturing the transformed or transfected cell.

9. A polynucleotide as claimed in any one of claims 1 to 4, an expression cassette as claimed in claim 5 an expression vector as claimed in claim 6 or a host cell as claimed in claim 7 for use in medical therapy.

## Revendications

1. Polynucléotide cloné ayant la fonction d'une séquence de régulation de la transcription (trs, transcriptional regulatory sequence) et comprenant :
(a) un fragment polynucléotidique ayant au moins 70 % d'identité avec le polynucléotide de SEQ ID N° 2 ;
(b) un polynucléotide qui est complémentaire du polynucléotide de (a) ; ou
(c) un polynucléotide comprenant au moins 15 bases séquentielles du polynucléotide de (a) ou (b).

2. Polynucléotide selon la revendication 1 dans lequel le fragment polynucléotidique présente au moins 80 % d'identité avec le polynucléotide de SEQ ID N° 2.

3. Polynucléotide selon la revendication 1 ou 2 dans lequel le fragment polynucléotidique présente au moins 90 % d'identité avec le polynucléotide de SEQ ID N° 2.

4. Polynucléotide selon la revendication 1, 2 ou 3 comprenant le polynucléotide de SEQ ID N° 2.

5. Cassette d'expression comprenant un polynucléotide selon l'une quelconque des revendications précédentes et un polynucléotide qui y soit lié de manière fonctionnelle codant un polypeptide hétérologue.

6. Vecteur d'expression comprenant la cassette d'expression selon la revendication 5.

7. Cellule hôte comprenant un vecteur selon la revendication 6.

8. Processus pour produire un polypeptide, lequel processus comprend la transformation ou la transfection d'une cellule avec un vecteur selon la revendication 6 et la mise en culture de la cellule transformée ou transfectée.

9. Polynucléotide selon l'une quelconque des revendications 1 à 4, cassette d'expression selon la revendication 5, vecteur d'expression selon la revendication 6, ou cellule hôte selon la revendication 7, destinés à être utilisés dans un traitement médical.

## Patentansprüche

1. Kloniertes Polynukleotid mit der Funktion einer transkriptionalen regulatorischen Sequenz (trs), das folgendes umfaßt:
(a) ein Polynukleotidfragment mit wenigstens 70 % Identität mit dem Polynukleotid von SEQ ID NO: 2:
(b) ein Polynukleotid, das komplementär zum Polynukleotid aus (a) ist; oder
(c) ein Polynukleotid, das wenigstens 15 aufeinanderfolgende Basen des Polynukleotids aus (a) oder (b) umfaßt.

2. Polynukleotid gemäß Anspruch 1, worin das Polynukleotidfragment wenigstens 80 % Identität mit dem Polynukleotid von SEQ ID NO: 2 hat.

3. Polynukleotid gemäß Anspruch 1 oder 2, worin das Polynukleotidfragment wenigstens 90 % Identität mit dem Polynukleotid von SEQ ID NO: 2 hat.

4. Polynukleotid gemäß Anspruch 1, 2 oder 3, das das Polynukleotid von SEQ ID NO: 2 umfaßt.

5. Expressionskassette, die ein Polynukleotid gemäß einem der vorhergehenden Ansprüche und ein operativ daran gebundenes Polynukleotid, das ein heterologes Polypeptid codiert, umfaßt.

6. Expressionsvektor, der die Expressionskassette gemäß Anspruch 5 umfaßt.

7. Wirtszelle, die einen Vektor gemäß Anspruch 6 umfaßt.

8. Verfahren zur Herstellung eines Polypeptids, wobei das Verfahren das Transformieren oder Transfizieren einer Zelle mit einem Vektor gemäß Anspruch 6 und das Kultivieren der transformierten oder transfizierten Zelle umfaßt.

9. Polynukleotid gemäß einem der Ansprüche 1 bis 4, Expressionskassette gemäß Anspruch 5, Expressionsvektor gemäß Anspruch 6 oder Wirtszelle gemäß Anspruch 7 zur Verwendung in der medizinischen Therapie.
